# EUROPÄISCHE PATENTSCHRIFT

(11) **EP 1 188 042 B1**
(45) Veröffentlichungstag und Bekanntmachung des Hinweises auf die Patenterteilung: **23.11.2005**
(21) Anmeldenummer: 00945639.3
(22) Anmeldetag: 10.06.2000
(51) Int. Cl.: G01N 21/75, G01N 33/543, G01N 21/63

(54) **VERFAHREN UND SONDE ZUR IDENTIFIZIERUNG EINES POLYMERS**
METHOD AND PROBE FOR IDENTIFYING A POLYMER
PROCEDE ET SONDE D'IDENTIFICATION D'UN POLYMERE

(30) Priorität: 14.06.1999 DE 19927051
(43) Veröffentlichungstag der Anmeldung: 20.03.2002
(73) Patentinhaber: November Aktiengesellschaft Gesellschaft für Molekulare Medizin, 91056 Erlangen (DE)
(72) Erfinder: BERTLING, Wolf, D-91056 Erlangen (DE); HASSMANN, Jörg, D-91052 Erlangen (DE)
(74) Vertreter: Gassner, Wolfgang
(86) Internationale Anmeldenummer: PCT/DE2000/001945
(87) Internationale Veröffentlichungsnummer: WO 2000/077496

(56) Entgegenhaltungen:
- EP-A- 0 762 122
- WO-A-97/04129
- WO-A-98/48275
- DE-A- 19 621 312
- FR-A- 2 762 394
- US-A- 4 687 732
- US-A- 5 686 071
- LECKBAND D.: 'The surface force apparatus - a tool for probing molecular protein interactions' NATURE Bd. 17, Nr. 6541, 17 August 1995, Seiten 617 - 618, XP000525968

## Beschreibung

Die Erfindung betrifft ein Verfahren und eine Sonde zur Identifizierung eines an einer elektromagnetische Wellen reflektierenden ersten Phase gebundenen ersten Polymers.

Aus der WO 98/48275 ist ein optischer Sensor bekannt, mit dem Nukleinsäuren, Proteine und deren Liganden erfaßt werden können. Zum Nachweis wird der optische Sensor z.B. in eine nukleinsäureenthaltende Lösung getaucht. Nach Spülen und Trocknen des Sensors kann dessen optische Eigenschaft ermittelt werden. - Das Verfahren unter Verwendung des bekannten Sensors erfordert mehrere Schritte; es ist zeitaufwendig.

Aus der WO 97/04129 ist ein verfahren zum Nachweis von Nukleinsauresequenzen bekannt, dabei ist eine erste Nukleinsäuresequenz an einer festen Oberfläche immobilisiert. Die Hybridisierung mit einer zweiten komplementären Nukleinsäuresequenz wird mittels oberflächensensitiver Nachweisverfahren unter bestimmten Bedingungen erfaßt.

Die WO 91/02981 beschreibt ein Verfahren zum Nachweis eines Analyten unter Verwendung der Oberflächenplasmonenresonanzspektroskopie. Auch dabei ist ein Analyt an einer Metalloberfläche immobilisiert. Zum Nachweis muß der Analyt wiederum mit einer Lösung in Kontakt gebracht werden.

Ferner ist aus der US 5,485,277 ein Sensor zur Durchführung der Oberflächenplasmonenresonanzspektroskopie bekannt. Der Sensor weist einen planaren Wellenleiter mit einer Vielzahl von Reflektorflächen auf.

Aus der US 4, 687, 732 ist ein Verfahren bekannt, bei dem ein für ein bestimmtes Target-Molekül spezifisches Detektionsmolekül vorgesehen ist. Das Detektionsmolekül ist kovalent an ein Polymer gebunden. Das Polymer erzeugt beim Binden des Target-Moleküls an das Detektionsmolekül ein optisch erfassbares Signal. Das Target-Molekül liegt dabei in einer Lösung vor.

Die EP-A-0 762 122. beschreibt einen optischen Festphasensensor, bei dem zur spezifischen Erkennung von Analyten der Förster-Energie-Transfer zwischen zwei Fluoreszenzstoffen ausgenutzt wird.

Aufgabe der Erfindung ist es, die Nachteile des Stands der Technik zu beseitigen. Es sollen insbesondere ein Verfahren und eine Vorrichtung angegeben werden, mit denen an einer festen Phase gebundene Polymere, insbesondere biochemische Moleküle, schnell und einfach nachgewiesen werden können.

Diese Aufgabe wird durch die Merkmale der Ansprüche 1 und 15 gelöst. Zweckmäßige Weiterbildungen der Erfindung ergeben sich aus den Merkmalen der Ansprüche 2 bis 14 und 16 bis 29.

Nach Maßgabe der Erfindung ist ein Verfahren zur Identifizierung eines an einer elektromagnetische Wellen reflektierenden ersten festen Phase gebundenen ersten Polymers mit folgenden Schritten vorgesehen:
a) Inkontaktbringen des ersten Polymers mit einem zum ersten polymer affinen zweiten Polymer, das über metallische Cluster an eine feste für elektromagnetische Wellen durchlässige zweite Phase gebunden ist,
b) Durchstrahlen der zweiten Phase mit elektromagnetischen Wellen und
c) Erfassen der Änderung der Eigenschaften der reflektierten elektromagnetischen Wellen.

Nach dem erfindungsgemäßen Verfahren muß das nachzuweisende Polymer, z.B. ein biochemisches Molekül, nicht unbedingt in Lösung vorliegen. Es kann z.B. auch zu Markierungszwecken an einem Festkörper, wie einer Banknote, gebunden sein. Durch einfaches Inkontaktbringen der zweiten für elektromagnetische Wellen durchlässigen Phase und Messung der optischen Eigenschaften des reflektierten Lichts kann sofort ermittelt werden, ob das nachzuweisende Polymer an der ersten festen Phase gebunden ist. Das Verfahren ist schnell und einfach durchführbar.

Vorteilhafterweise werden als elektromagnetische Wellen Licht, vorzugsweise LASER, verwendet. Die Eigenschaften von reflektiertem Licht können besonders einfach ermittelt werden.

Als Änderung der Eigenschaft kann die Absorption in einem vorgegebenen Spektrum vor und/öder nach dem Inkontaktbringen des ersten und des zweiten Polymers gemessen werden. Ferner kann bei Verwendung von monochromatischem Licht als Änderung der Eigenschaft die spektrale Verschiebung gemessen werden. Ferner kann als Änderung der Eigenschaft die zeitliche Änderung der Absorption und/oder Reflexion beim Inkontaktbringen und/oder Trennen des ersten und zweiten Polymers gemessen werden. Die Änderung der Eigenschaft kann unter mehreren voneinander verschiedenen Einfallswinkeln gemessen werden- Es ist denkbar, auch andere Änderungen der Eigenschaften des reflektierten Lichts zu messen. Die Wahl, welche Änderung erfaßt wird, richtet sich nach den jeweiligen Gegebenheiten.

Die metallischen Cluster können unmittelbar auf die zweite Phase aufgedampft oder auch über eine aus dem zweiten Polymer gebildete Schicht an die zweite Phase gebunden sein. Auf der Oberfläche der zweiten Phase kann eine aus dem zweiten Polymer gebildete Schicht aufgebracht sein. Bei dem zweiten Polymer kann es sich z.B. um Poly-(D-glucosamin) handeln. Zwischen der auf der Oberfläche aufgebrachten Schicht und der mit den metallischen Clustern verbundenen Schicht kann mindestens eine aus dem ersten Polymer gebildete Schicht zwischengeschaltet sein. Es kann auch eine aus dem ersten und dem zweiten Polymer gebildete Schichtfolge zwischengeschaltet sein. Das Vorsehen einer solchen Schichtfolge trägt zur Ausbildung besonders deutlich und schnell identifizierbarer Signale bei. Auf der Oberfläche der ersten Phase kann eine aus dem ersten Polymer gebildete Schicht aufgebracht sein. Die Oberfläche kann aus einer Oxidschicht des Metalls, z.B. einer Aluminiumoxidschicht, gebildet sein. Auf der Oberfläche kann auch eine aus dem ersten und dem zweiten Polymer gebildete Schichtfolge vorgesehen sein, wobei die äußerste Schicht und die auf der Oberfläche gebundene Schicht aus dem ersten Polymer gebildet sein kann. Beim ersten Polymer kann es sich z.B. um Polyacrylsäure (=PAS) handeln.

Als erstes und/oder zweites Polymer wird zweckmäßigerweise ein Polynukleotidmolekül wie DNA, RNA, ss-DNA, ss-RNA oder synthetische Analoga davon, Protein, Peptid, Peptidnukleinsäure (PNA) oder ein Ligand davon, oder Polyacrylsäure, Poly-(D-glucosamin), Polyethylenimin verwendet. Grundsätzlich eignen sich insbesondere alle biochemischen Moleküle mit rekombinanten Eigenschaften.

Beim Schritt a) kann auch mindestens ein weiteres an die erste Phase gebundenes Polymer mit dem zweiten Polymer in Kontakt gebracht werden. Das ermöglicht es, gleichzeitig eine Mehrzahl an Identifizierungsversuchen durchzuführen. So kann z.B- aus den Polymeren ein Strichcode oder ein ähnliches Muster auf der Oberfläche z.B. der ersten Phase gebildet sein.

Bei einer Sonde zur Identifizierung einer Markierung mit einer elektromagnetische Wellen reflektierenden ersten festen Phase (5), an die ein erstes Polymer (4, 7) gebunden ist, ist erfindungsgemäß vorgesehen, dass an eine für elektromagnetische Wellen durchlässige zweite Phase (1) ein zum ersten Polymer (4, 7) affines zweite Polymer (3, 8) über metallische Cluster (2) gebunden ist, wobei bei einem Kontakt zwischen dem ersten (4, 7) und dem zweiten Polymer (3, 8) eine Affinität zwischen dem ersten (4, 7) und zweiten Polymer (3, 8) durch die zweite Phase (1) hindurch als Änderung der optischen Eigenschaften von an der ersten Phase (5) reflektiertem Licht erfassbar ist.

Die erfindungsgemäße Sonde erlaubt eine schnelle und einfache Identifizierung eines ersten Polymers. Ein Spülen und Trocknen der Vorrichtung ist zur Messung der optischen Eigenschaften der verwendeten elektromagnetischen Wellen nicht erforderlich. Unter Affinität wird verstanden, daß die Polymere durch Wechselwirkungen einen gebundenen oder assoziierten Zustand einnehmen können. Solche Bindungen können z.B. Wasserstoffbrückenbindungen, ionische, hydrophobe oder kovalente Bindungen sein. Ferner kommen Komplex-Bindungen oder durch sterische Einflüsse hervorgerufene Bindungen in Betracht. Z.B. werden die Stränge zueinander komplementärer Biomoleküle, wie DNA, als affin angesehen; sie können hybridisieren.

Es hat sich als zweckmäßig erwiesen, die metallischen Cluster aus Silber, Gold, Aluminium, Kupfer oder Indium zu bilden. An solche Metalle binden Polymere besonders gut.

Als elektromagnetische wellen kann Licht, vorzugsweise LASER, verwendet werden. Vorteilhafterweise ist die zweite Phase aus einem transparenten Material, wie Kunststoff oder Glas, hergestellt. Das erste und/oder zweite Polymer kann DNA, RNA, Protein, Peptid, Peptidnukleinsäure oder ein Ligand davon, oder Polyacrylsäure, Poly-(D-glucosamin), Polyethylenimin sein. Als Polymer kann aber auch ss-DNA, ss-RNA oder synthetische Analoga davon verwendet werden.

Als weiterer Bestandteil der Sonde bzw. Vorrichtung kann eine Einrichtung zur Bestimmung der optischen Eigenschaften des reflektierten Lichts vorgesehen sein. Mittels der Einrichtung kann die Absorption in einem vorgegebenen Spektrum vor und/oder nach dem Inkontaktbringen des ersten und des zweiten Polymers meßbar sein. Ferner kann mittels der Einrichtung die spektrale Verschiebung des reflektierten Lichts meßbar sein.

Zweckmäßigerweise ist mittels der Einrichtung die optische Eigenschaft unter mehreren voneinander verschiedenen Einfallswinkeln meßbar.

Die metallischen Cluster können über eine aus dem zweiten Polymer gebildete Schicht an die zweite Phase gebunden sein. Auf der Oberfläche der zweiten Phase kann ferner eine aus dem zweiten Polymer gebildete Schicht aufgebracht sein. Zweckmäßigerweise sind außerdem zwischen der auf der Oberfläche vorgesehenen Schicht und der mit den metallischen Clustern verbundenen Schicht mindestens eine aus dem ersten Polymer gebildete Schicht zwischengeschaltet ist. Auf der Oberfläche der ersten Phase kann eine aus dem ersten Polymer gebildete Schicht und/oder auf der auf der Oberfläche vorgesehenen Schicht kann eine aus dem zweiten Polymer gebildete Schicht aufgebracht sein. Der beschrieben Schichtkomplex ermöglicht eine Markierung und eine einfache und schnelle Identifizierung der Markierung.

Nachfolgend wird die Erfindung anhand der in der Zeichnung dargestellten Ausführungsbeispiel näher erläutert. Es zeigen:
- Fig. 1: eine schematische Ansicht einer Vorrichtung ,
- Fig. 2: die Vorrichtung nach Fig. 1 im nichthybridisierten Fall,
- Fig. 3: die Vorrichtung nach Fig. 1 im hybridisierten Fall,
- Fig. 4: eine schematische Ansicht einer weiteren Vorrichtung und
- Fig. 5: den Nachweis einer Markierung.

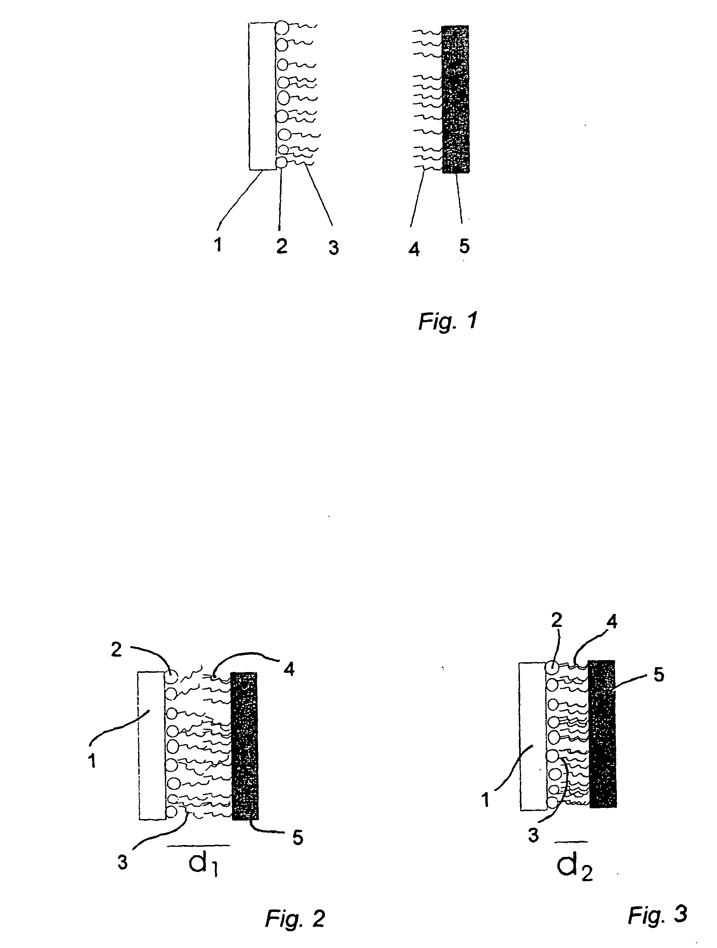

In den Fig. 1 - 3 ist eine zweite feste Phase beispielsweise aus einem Glasträger 1 hergestellt. Auf einer Oberfläche des Glasträgers 1 befinden sich metallische Cluster 2, z.B. Goldcluster. An die Cluster 2 gebunden ist als zweites Polymer eine einzelsträngige DNA 3. Als erstes Polymer ist eine weitere einzelsträngige DNA 4 an eine Metallfolie 5 gebunden. Die Metallfolie 5 kann wiederum beispielsweise zu Markierungszwecken an Banknoten angebracht sein (hier nicht dargestellt).

Sofern die DNA 3 und die weitere DNA 4 in Kontakt gebracht werden, sind zwei Fälle zu unterscheiden:

Im ersten in Fig. 2 gezeigten Fall ist die DNA 3 nicht komplementär zur weiteren DNA 4. Es findet keine Hybridisierung statt. Zwischen der durch die Cluster 2 gebildeten Schicht und der Metallfolie 5 stellt sich ein erster Äbstand d₁ ein.

Im zweiten in Fig. 3 gezeigten Fall ist die DNA 3 komplementär zur weiteren DNA 4. Die DNA 3 und die weitere DNA 4 hybridisieren. Es stellt sich ein kleinerer zweiter Abstand d₂ zwischen der durch die Cluster 2 gebildeten Schicht und der Metallfolie 5 ein.

Ein durch den Glasträger 1 einfallender LaserstrahJ. (hier nicht dargestellt) wird an der Metallfolie 5 reflektiert. Die Eigenschaften des reflektierten Lichts hängen vom Abstand d₁, d₂ der durch die Cluster 2 gebildeten Schicht von der Metallfolie 5 ab. So ändert sich beispielsweise die Absorption. Durch Messung der Absorption kann auf einfache Weise ermittelt werden, ob eine Hybridisierung vorliegt oder nicht. Das ermöglicht die Identifizierung der ersten Polymers 4.

Zur Herstellung der in den Fig. 1 - 3 gezeigten optischen Sonde wird ein Glasträger 1 mit Gold bedampft. Dazu wird der Glasträger 1 in eine Vakuumkammer gehängt, in welcher gleichzeitig eine Goldfolie plaziert ist. Nach Abpumpen auf 10⁻² mbar (= 1 Pa) und anschließendes Spülen der Vakuumkammer mit Argongas wird der Druck in der Vakuumkajnmer auf etwa 10⁻¹ mbar (= 10 Pa) eingestellt. Danach wird ein Plasma gezündet, durch welches Goldatome aus der Folie gesputtert werden. Die Goldatome schlagen sich an der Oberfläche des Glasträgers 1 nieder. Bei einem Sputterstrom von 40 mA bildet sich nach etwa 10 Sekunden ein Goldfilm mit einer Massendicke von 5 nm. Danach wird der Goldfilm bei etwa 200° C ausgeheilt. Es bilden sich runde Goldcluster 2, die für den erwünschten Farbverstärkungseffekt geeignet sind. Anschließend wird der mit Goldclustern 2 beschichtete Glasträger 1 in eine Lösung getaucht, die Oligonukleotide 3 enthält, welche an ihrem 5'-Ende mit einer Thiolgruppe versehen sind. Die Oligonukleotide 3 lagern sich unter Bildung einer Thiolbindung an die Goldcluster an.

Zur Herstellung der mit dem Bezugszeichen 4 und 5 bezeichneten Probe wird beispielsweise ein Aluminiumsubstrat 5 hergenommen. Durch eine definierte elektrochemische Oxidation des Aluminiumsubstrats 5 beispielsweise in 5 Siger Oxalsäure bei 300 mA und etwa 50 V werden durch stufenweise tieferes Eintauchen des Aluminiumsubstrats 5 in die Lösung Oxidschichten unterschiedlicher Dicke erzeugt. Diese Oxidschichten weisen infolge von Interferenzeffekten unterschiedliche Farben auf.

Zur kovalenten Ankopplung von weiteren Oligonukleotiden 4, welche an ihrem 5'-Ende mit Aminogruppen versehen sind, werden die Oxidschichten mit einer eine freie Aminogruppe tragenden Schicht überzogen. Dazu wird das Aluminiumsubstrat 5 für etwa 30 Minuten in eine etwa 10 %ige wäßrige Aminopropyltriethoxysilan-Lösung bei einem pH-Wert von etwa 9 getaucht. Anschließend wird das Aluminiumsubstrat mit Wasser gespült und in einem Trockenofen bei etwa 80° C eine Stunde getrocknet.

Die so erzeugten silanisierten Aluminiumsubstrate 5. werden dann in eine 2,5 %ige Glutardialdehydlösung, welche 50 mMol/l NaCNBH₃ enthält, für 12 Stunden inkubiert. Anschließend wird gründlich rait Wasser gewaschen.

Zur Anbinding der weiteren Oligonukleotide 4 werden die Aluminiumsubstrate 5 über Nacht bei 4° C in einer wäßrigen Pufferlösung inkubiert, welche weitere Oligonukleotide 4 in einer Konzentration von 1 µMol/l, 0,1 x PBS-Puffer und 50 mMol/l NaCNB₃ enthalten. Danach werden die Aluminiumsubstrate 5 nochmals gründlich mit Wasser gespült. Sie weisen dann kovalent gebundene weitere Oligonukleotide 4 an ihrer Oberfläche auf.

Im Hinblick auf weitere Einzelheiten, insbesondere die Größe der Cluster 2 sowie die Abstandsparameter, wird auf die wo 98/48275 verwiesen, deren diesbezüglicher Offenbarungsgehalt hiermit einbezogen wird.

Zum Nachweis einer Hybridisierung zwischen der DNA 3 bzw. dem Detektoroligonukleotid und der weiteren DNA 4 bzw. dem Markierungsoligonukleotid werden der Glasträger 1 und die Metallfolie 5, z.B. das nach dem vorgenannten Verfahren hergestellte Aluminiumsubstrat, aufeinander gepreßt. Infolge unterschiedlicher Abstände zwischen der reflektierenden Aluminiumoberfläche und den Goldclustern 2 sind charakteristische Farbmuster erkennbar. Diese lassen darauf schließen, ob eine Hybridisierung zwischen dem Markierungs- und dem Detektoroligonukleotid vorliegt oder nicht.

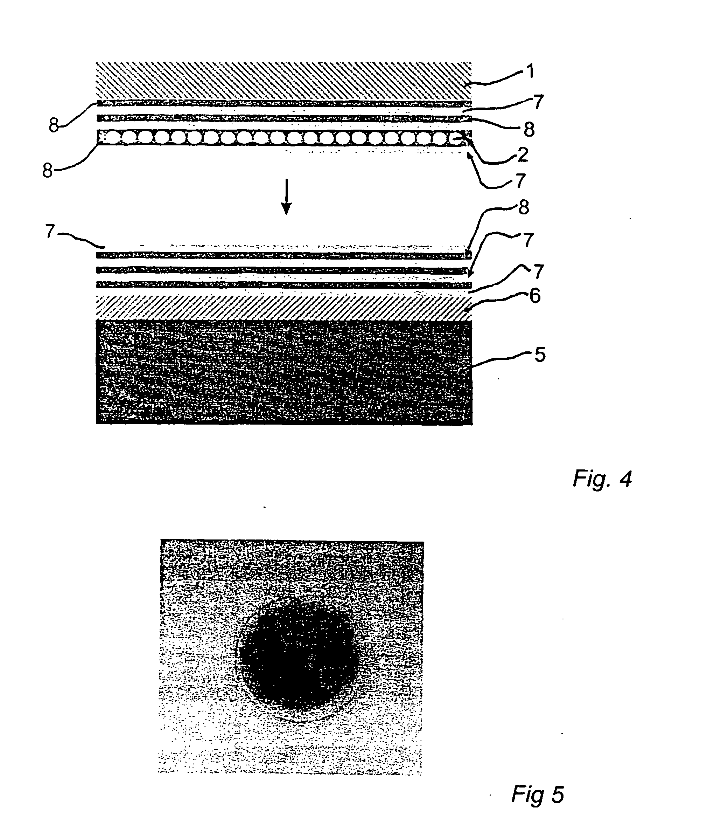

In Fig. 4 ist ein weiteres Ausführungsbeispiel der Erfindung gezeigt. Auf einer aus Aluminium hergestellten Metallfolie 5 befindet sich eine Aluminiumoxidschicht 6. Die Aluminiumoxidschicht 6 ist überdeckt von einer PAS-Schicht 7. Darüber folgt eine Poly-(D-gluaosamin)-Schicht 8. Es sind mehrere solcher aus PAS und Poly-(D-glucosamin) bestehende Schichtfolgen vorgesehen. Die oberste Schicht wird durch eine PAS-Schicht 7 gebildet.

Die Sonde besteht aus einem Glasträger 1, welcher das Substrat bildet. Auf dessen Oberfläche ist eine Poly-(D-glucosamin)-Schicht 8 vorgesehen. Darüber lagert eine PAS-Schicht 7. Es sind mehrere aus Poly-(D-glucosamin) 8 und PAS 7 bestehende Schichtabfolgen vorgesehen. Auf einer Poly-(D-glucosamin)-Schicht 8 in der Nähe der Oberfläche sind Goldcluster 2 gebunden. Darüber lagert eine weitere Poly-(D-glucosamin)-Schicht 8. Diese ist zu Testzwecken abschnittsweise überlagert von einer PAS-Schicht 7.

Zur Herstellung der beschichteten Aluminiumfolie 5 wird diese durch abwechselndes Eintauchen in eine Polyacrylsäure enthaltende Lösung sowie eine Poly-(D-glucosamin)-Lösung beschichtet. Die Beschichtungszeit hat jeweils 15 Minuten und die Konzentration der Lösungen 0,5 g/l betragen. Zur Beschichtung mit den Goldclustern 2 wird die Sonde in eine Lösung getaucht, die etwa 0,4 % Goldcluster mit einem Durchmesser von 25 nm enthält. Die Goldcluster 2 binden durch adsorptive Kräfte. Anschließend wird die Sonde nochmals in eine Poly-(D-glucosamin)-Lösung getaucht, so daß die Goldcluster in die Schichtabfolge eingepackt sind. Zum Funktionsnachweis des Verfahrens ist die Sonde abschließend zur Hälfte in PAS getaucht worden. Die Sonde ist so abschnittsweise mit einer PAS-Schicht 7 überzogen.

Bei der vorbeschriebenen Anordnung ist der Anschaulichkeit halber die Markierung auf der Sonde aufgebracht. In der Praxis wird man die Markierung in analoger Weise auf der Probe vorsehen.

Fig. 5 zeigt den Kontakt der kreisförmigen. Sonde mit dem beschichteten Aluminiumoxidsubstrat 5. Darin ist eine Grenzlinie erkennbar, welche von links oben nach rechts unten verläuft. Der helle Bereich entspricht der mit einer Poly-(D-glucosamin)-Schicht 8 beschichtete Bereich der Sonde. Der dunkle Bereich entspricht dem Abschnitt der Sonde, welcher an seiner Oberfläche die PAS-Schicht 7 tragt. Infolge der repulsiven Wechselwirkung der oberflächlichen PAS-Schicht 7 der Sonde mit der PAS-Schicht 7 des Substrats kommt es zu einer Lichtreflexion, welche anders ist, als die im Kontaktbereich der Poly-(D-glucosamin)-Schicht 8 der Sonde, welche attrakiv mit der PAS-Schicht 7 des Aluminiumsubstrats 5 wechselwirkt. Es ist also klar unterscheidbar, ob eine auf dem Aluminiumsubstrat 5 aufgebrachte, z.B. aus PAS oder einem anderen Polymer, insbesondere einem Biopolymer, gebildete Markierung affin ist zu der Sonde.

## Patentansprüche

1. Verfahren zur Identifizierung eines an einer elektromagnetische Wellen reflektierenden ersten festen Phase (5) gebundenen ersten Polymers (4, 7) mit folgenden Schritten:
a) Inkontaktbringen des ersten Polymers (4, 7) mit einem zum ersten Polymer (4, 7) affinen zweiten Polymer (3, 8), das über metallische Cluster (2) an eine feste für elektromagnetische Wellen durchlässige zweite Phase (1) gebundenen ist,
b) Durchstrahlen der zweiten Phase (1) mit elektromagnetischen Wellen und
c) Erfassen der Änderung der Eigenschaften der reflektierten elektromagnetischen Wellen.

2. Verfahren nach Anspruch 1, wobei als elektromagnetische Wellen Licht, vorzugsweise erzeugt durch einen LASER, verwendet werden.

3. Verfahren nach einem der vorhergehenden Ansprüche, wobei als Änderung der Eigenschaft die Absorption in einem vorgegebenen Spektrum vor und/oder nach dem Inkontaktbringen des ersten (4, 7) mit dem zweiten Polymers (3, 8) gemessen wird.

4. Verfahren nach einem der vorhergehenden Ansprüche, wobei bei Verwendung von monochromatischem Licht als Änderung der Eigenschaft die spektrale Verschiebung gemessen wird.

5. Verfahren nach einem der vorhergehenden Ansprüche, wobei als Änderung der Eigenschaft die zeitliche Änderung der Absorption und/oder Reflexion beim Inkontaktbringen und/oder Trennen des ersten (4, 7) und zweiten Polymers (3, 8) gemessen wird.

6. Verfahren nach einem der vorhergehenden Ansprüche, wobei die Änderung der Eigenschaft unter mehreren voneinander verschiedenen Einfallswinkeln gemessen wird.

7. Verfahren nach einem der vorhergehenden Ansprüche, wobei die metallischen Cluster (2) unmittelbar auf die zweite Phase (1) aufgedampft oder über eine aus dem zweiten Polymer (3, 8) gebildete Schicht an die zweite Phase (1) gebunden sind.

8. Verfahren nach Anspruch 7, wobei auf der Oberfläche der zweiten Phase (1) eine aus dem zweiten Polymer (3, 8) gebildete Schicht aufgebracht ist.

9. Verfahren nach Anspruch 8, wobei zwischen der auf der Oberfläche aufgebrachten Schicht und der mit den metallischen Clustern (2) verbundenen Schicht mindestens eine aus dem ersten Polymer (4, 7) gebildete Schicht zwischengeschaltet ist.

10. Verfahren nach einem der vorhergehenden Ansprüche, wobei auf der Oberfläche der ersten Phase (5) eine aus dem ersten Polymer (4, 7) gebildete Schicht aufgebracht ist.

11. Verfahren nach Anspruch 10, wobei auf der Oberfläche eine aus dem ersten (4, 7) und dem zweiten Polymer (3, 8) gebildete Schichtfolge aufgebracht ist.

12. Verfahren nach einem der vorhergehenden Ansprüche, wobei als erstes (4, 7) und/oder zweites Polymer (3, 8) DNA, RNA, ss-DNA, ss-RNA oder synthetische Analoga davon, Protein, Peptid, Peptidnukleinsäure (PNA) oder ein Ligand davon, oder Polyacrylsäure, Polyethylenimin, Poly-(D-glucosamin) verwendet wird/werden.

13. Verfahren nach einem der vorhergehenden Ansprüche, wobei beim Schritt a) mindestens ein weiteres an die erste Phase (5) gebundenes Polymer mit dem zweiten Polymer (3, 8) in Kontakt gebracht wird.

14. Verfahren nach Anspruch 13, wobei die Polymere (3, 4, 7, 8) an der ersten Phase (5) in Form eines Strichcodes aufgebracht sind.

15. Sonde zur Identifizierung einer Markierung mit einer elektromagnetische Wellen reflektierenden ersten festen Phase (5), an die ein erstes Polymer (4, 7) gebunden ist,
**gekennzeichnet durch**
eine für elektromagnetische Wellen durchlässigen zweiten Phase (1), an die ein zum ersten Polymer (4, 7) affines zweites Polymer (3, 8) über metallische Cluster (2) gebunden ist, wobei bei einem Kontakt zwischen dem ersten (4, 7) und dem zweiten Polymer (3, B) eine Affinität zwischen dem ersten (4, 7) und zweiten Polymer (3, 8) **durch** die zweite Phase (1) hindurch als Änderung der optischen Eigenschaften von an der ersten Phase (5) reflektiertem Licht erfassbar ist.

16. Vorrichtung nach Anspruch 15, wobei die metallischen Cluster (2) aus Silber, Gold, Aluminium, Kupfer oder Indium gebildet sind.

17. Vorrichtung nach Anspruch 15 oder 16, wobei die elektromagnetischen Wellen Licht, vorzugsweise erzeugt durch einen LASER, sind.

18. Vorrichtung nach einem der Ansprüche 15 bis 17, wobei die zweite Phase (1) aus einem transparenten Material, wie Kunststoff oder Glas, hergestellt ist.

19. Vorrichtung nach einem der Ansprüche 15 bis 18, wobei das erste (4, 7) und/oder zweite Polymer (3, 8) DNA, RNA, ss-DNA, ss-RNA oder synthetische Analoga davon, Protein, Peptid, Peptidnukleinsäure (PNA) oder ein Ligand davon, oder Polyacrylsäure, Poly-(D-glucosamin), Polyethylenimin ist.

20. Vorrichtung nach einem der Ansprüche 15 bis 19, wobei eine Einrichtung zur Bestimmung der optischen Eigenschaft des reflektierten Lichts vorgesehen ist.

21. Vorrichtung nach Anspruch 20, wobei mittels der Einrichtung die Absorption in einem vorgegebenen Spektrum vor und/oder nach dem Inkontaktbringen des ersten (4, 7) und des zweiten Polymers (3, 8) meßbar ist.

22. Vorrichtung nach einem der Ansprüche 20 oder 21, wobei mittels der Einrichtung die spektrale Verschiebung des reflektierten Lichts meßbar ist.

23. Vorrichtung nach einem der Ansprüche 15 bis 23, wobei mittels der Einrichtung die optische Eigenschaft unter mehreren voneinander verschiedenen Einfallswinkeln meßbar ist.

24. Vorrichtung nach einem der Ansprüche 15 bis 23, wobei die metallischen Cluster (2) über eine aus dem zweiten Polymer (3, 8) gebildete Schicht an die zweite Phase (1) gebunden sind.

25. Vorrichtung nach Anspruch 24, wobei auf der Oberfläche der zweiten Phase (1) eine aus dem zweiten Polymer (3, 8) gebildete Schicht aufgebracht ist.

26. Vorrichtung nach Anspruch 25, wobei zwischen der auf der Oberfläche vorgesehenen Schicht und der mit den metallischen Clustern (2) verbundenen Schicht mindestens eine aus dem ersten Polymer (4, 7) gebildete Schicht zwischengeschaltet ist.

27. Vorrichtung nach einem der Ansprüche 15 bis 26, wobei auf der Oberfläche der ersten Phase (5) eine aus dem ersten Polymer (4, 7) gebildete Schicht aufgebracht ist.

28. Vorrichtung nach Anspruch 27, wobei auf der auf der Oberfläche vorgesehenen Schicht eine aus dem zweiten Polymer (3, 8) gebildete Schicht aufgebracht ist.

29. Vorrichtung nach einem der vorhergehenden Ansprüche, wobei die Markierung an einen Festkörper, vorzugsweise eine Banknote, gebunden ist.

## Claims

1. A method for identifying a first polymer (4, 7) which is bound to a first phase (5) which reflects electromagnetic waves, which method has the following steps:
a) bringing the first polymer (4, 7) into contact to a second polymer (3, 8) which has affinity to the first polymer (4, 7) and which is bound, by way of metallic clusters (2), to a solid second phase (1) which is pervious to electromagnetic waves,
b) irradiating the second phase (1) with electromagnetic waves, and
c) detecting the change in the properties of the reflected electromagnetic waves.

2. The method as claimed in claim 1, **characterised in that** light, preferably light produced by a LASER, is used as the electromagnetic waves.

3. The method as claimed in either of the preceding claims, **characterised in that** the property change which is measured is the absorption in a predetermined spectrum before and/or after the first polymer (4, 7) has been brought into contact with the second polymer (3, 8).

4. The method as claimed in one of the preceding claims, **characterised in that**, when monochromatic light is used, the property change which is measured is the spectral shift.

5. The method as claimed in one of the preceding claims, **characterised in that** the property change which is measured is the chronological change in the absorption and/or reflection when the first polymer (4, 7) and the second polymer (3, 8) are brought into contact and/or separated.

6. The method as claimed in one of the preceding claims, **characterised in that** the property change is measured at several angles of incidence which differ from each other.

7. The method as claimed in one of the preceding claims, **characterised in that** the metallic clusters (2) are evaporation-coated directly onto the second phase (1) or are bound to the second phase (1) by way of a layer which is formed from the second polymer (3, 8).

8. The method as claimed in claim 7, **characterised in that** a layer formed from the second polymer (3, 8) is applied to the surface of the second phase (1).

9. The method as claimed in claim 8, **characterised in that** at least one layer formed from the first polymer (4, 7) is intercalated between the layer which is applied to the surface and the layer which is bonded to the metallic clusters (2).

10. The method as claimed in one of the preceding claims, **characterised in that** a layer formed from the first polymer (4, 7) is applied to the surface of the first phase (5).

11. The method as claimed in claim 10, **characterised in that** a layer sequence formed from the first polymer (4, 7) and the second polymer (3, 8) is applied to the surface.

12. The method as claimed in one of the preceding claims, **characterised in that** the first polymer (4, 7) and/or second polymer (3, 8) employ and/or employs DNA, RNA, ssDNA or ssRNA or synthetic analogs thereof, protein, peptide, peptide nucleic acid (PNA) or a ligand thereof, or polyacrylic acid, polyethylenimine or poly(D-glucosamine).

13. The method as claimed in one of the preceding claims, **characterised in that**, in the step denoted with the letter a, at least one other polymer, which is bound to the first phase (5), is brought into contact with the second polymer (3, 8).

14. The method as claimed in claim 13, **characterised in that** the polymers (3, 4, 7, 8) are applied to the first phase (5) in the form of a barcode.

15. Probe for identifying a label having a first solid phase (5) which reflects electromagnetic waves and a first polymer (4, 7) being bound to the first phase (5),
**characterised in**
a second phase (1) being previous to electromagnetic waves and a second polymer (3, 8) being bound by way of metallic clusters (2) to the second phase, wherein upon a contact between the first (4, 7) and the second polymer (3, 8) an affinity between the first (4, 7) and the second polymer (3, 8) can be observed through the second phase (1) as a change in the optical properties of light being reflected at the first phase (5).

16. The device as claimed in claim 15, **characterised in that** the metallic clusters (2) are formed from silver, gold, aluminium, copper or indium.

17. The device as claimed in claim 15 or 16, **characterised in that** the electromagnetic waves are light, preferably light produced by a LASER.

18. The device as claimed in one of claims 15 to 17, **characterised in that** the second phase (1) is produced from a transparent material, such as plastic or glass.

19. The device as claimed in one of claims 15 to 18, **characterised in that** the first polymer (4, 7) and/or the second polymer (3, 8) is/are DNA, RNA, ssDNA or ssRNA or synthetic analogs thereof, protein, peptide, peptidenucleic acid (PNA) or a ligand thereof, or polyacrylic acid, poly(D-glucosamine) or polyethylenimine.

20. The device as claimed in one of claims 15 to 19, **characterised in that** a contrivance for determining the optical property of the reflected light is provided.

21. The device as claimed in claim 20, **characterised in that** the contrivance can be used to measure the absorption in a predetermined spectrum before and/or after the first polymer (4, 7) and the second polymer (3, 8) are brought into contact.

22. The device as claimed in claim 20 or 21, **characterised in that** the contrivance can be used to measure the spectral shift of the reflective light.

23. The device as claimed in one of claims 15 to 23, **characterised in that** the contrivance can be used to measure the optical property at several angles of incidence which differ from each other.

24. The device as claimed in one of claims 15 to 23, **characterised in that** the metallic clusters (2) are bound to the second phase (1) by way of a layer which is formed from the second polymer (3, 8).

25. The device as claimed in claim 24, **characterised in that** a layer which is formed from the second polymer (3, 8) is applied to the surface on the second phase (1).

26. The device as claimed in claim 25, **characterised in that** at least one layer which is formed from the first polymer (4, 7) is intercalated between the layer provided on the surface and the layer which is bonded to the metallic clusters (2).

27. The device as claimed in one of claims 15 to 26, **characterised in that** a layer which is formed from the first polymer (4, 7) is applied to the surface of the first phase (5).

28. The device as claimed in claim 27, **characterised in that** a layer which is formed from the second polymer (3, 8) is applied to the layer which is provided on the surface.

29. The device as claimed in one of the preceding claims, **characterised in that** the label is bound at a solid, preferably at a banknote.

## Revendications

1. Procédé pour l'identification d'un premier polymère (4, 7) lié à une première phase fixe (5) réfléchissant des ondes électromagnétiques selon les opérations suivantes :
a) Mise en contact du premier polymère (4, 7) avec un deuxième polymère (3, 8) affine au premier polymère (4, 7) qui est lié par des clusters métalliques (2) à une deuxième phase fixe perméable (1) aux ondes électromagnétiques,
b) Pénétration de la deuxième phase (1) avec des ondes électromagnétiques et
c) Saisie de la modification des propriétés des ondes électromagnétiques réfléchies

2. Procédé selon la revendication 1, de la lumière générée de préférence par un LASER étant utilisée comme ondes électromagnétiques.

3. Procédé selon l'une des revendications précédentes, l'absorption étant mesurée comme modification de la propriété dans un spectre défini avant et/ou après la mise en contact du premier (4, 7) avec le deuxième polymère (3, 8).

4. Procédé selon l'une des revendications précédentes, le déplacement spectral étant mesuré comme modification de la propriété en cas d'utilisation de lumière monochromatique.

5. Procédé selon l'une des revendications précédentes, la modification temporelle de l'absorption et/ou de la réflexion lors de la mise en contact et/ou de la séparation du premier (4, 7) et du deuxième polymère (3, 8) étant mesurée comme modification de la propriété.

6. Procédé selon l'une des revendications précédentes, la modification de la propriété étant mesurée sous plusieurs angles d'incidence différents les uns des autres.

7. Procédé selon l'une des revendications précédentes, les clusters métalliques (2) étant métallisés sous vide directement sur la deuxième phase (1) ou liés à la deuxième phase (1) via une couche formée à partir du deuxième polymère (3, 8).

8. Procédé selon la revendication 7, une couche formée à partir du deuxième polymère (3, 8) étant appliquée sur la surface de la deuxième phase (1).

9. Procédé selon la revendication 8, au moins une couche formée à partir du premier polymère (4, 7) étant insérée entre la couche appliquée sur la surface et la couche reliée aux clusters métalliques (2).

10. Procédé selon l'une des revendications précédentes, une couche formée à partir du premier polymère (4, 7) étant appliquée sur la surface de la première phase (5).

11. Procédé selon la revendication 10, une suite de couches formée à partir du premier (4, 7) et du deuxième polymère (3, 8) étant appliquée sur la surface.

12. Procédé selon l'une des revendications précédentes, un ADN, ARN, ss-ADN, ss-ARN ou des analogues synthétiques dont protéine, peptide, acide nucléique peptidique (PNA) ou un de leur ligand, ou acide polyacrylique, polyéthylène imine, poly-(D-glucosamine) est/sont utilisé(s) comme premier (4, 7) et/ou deuxième polymère (3, 8).

13. Procédé selon l'une des revendications précédentes, au moins un autre polymère lié à la première phase (5) étant mis en contact avec le deuxième polymère (3, 8) à l'opération a).

14. Procédé selon la revendication 13, les polymères (3, 4, 7, 8) étant appliqués sur la première phase (5) sous forme de code barre.

15. Sonde pour l'identification d'un marquage avec une première phase fixe (5) réfléchissant des ondes électromagnétiques à laquelle est lié un premier polymère (4, 7),
**caractérisé en ce qu'**
une deuxième phase (1) perméable aux ondes électromagnétiques à laquelle est lié par l'intermédiaire des clusters métalliques (2) un deuxième polymère (3, 8) affine au premier polymère (4, 7), en cas de contact entre le premier (4, 7) et le deuxième polymère (3, 8) une affinité entre le premier (4, 7) et le deuxième polymère (3, 8) pouvant être détectée par la deuxième phase (1) comme modification des propriétés optiques de la lumière réfléchie à la première phase (5).

16. Dispositif selon la revendication 15, les clusters métalliques (2) étant en argent, or, aluminium, cuivre ou indium.

17. Dispositif selon la revendication 15 ou 16, les ondes électromagnétiques étant de la lumière générée de préférence par un LASER.

18. Dispositif selon l'une des revendications 15 à 17, la deuxième phase (1) étant en matière transparente, telle que du plastique ou du verre.

19. Dispositif selon l'une des revendications 15 à 18, le premier (4, 7) et/ou le deuxième polymère (3, 8) étant un ADN, ARN, ss-ADN, ss-ARN ou des analogues synthétiques, protéine, peptide, acide nucléique peptidique (PNA) ou un de leur ligand, ou acide polyacrylique, poly-(D-glucosamine), polyéthylène imine.

20. Dispositif selon l'une des revendications 15 à 19, un équipement étant prévu pour déterminer la propriété optique de la lumière réfléchie.

21. Dispositif selon la revendication 20, l'équipement permettant de mesurer l'absorption dans un spectre prédéfini avant et/ou après la mise en contact du premier (4, 7) et du deuxième polymère (3, 8).

22. Dispositif selon l'une des revendications 20 ou 21, l'équipement permettant de mesurer le déplacement spectral de la lumière réfléchie.

23. Dispositif selon l'une des revendications 15 à 23, l'équipement permettant de mesurer la propriété optique sous plusieurs angles d'incidence différents les uns des autres.

24. Dispositif selon l'une des revendications 15 à 23, les clusters métalliques (2) étant liés à la deuxième phase (1) par l'intermédiaire d'une couche formée à partir du deuxième polymère (3, 8).

25. Dispositif selon la revendication 24, une couche formée à partir du deuxième polymère (3, 8) étant appliquée sur la surface de la deuxième phase (1).

26. Dispositif selon la revendication 25, au moins une couche formée à partir du premier polymère (4, 7) étant insérée entre la couche prévue sur la surface et la couche reliée aux clusters métalliques (2).

27. Dispositif selon l'une des revendications 15 à 26, une couche formée à partir du premier polymère (4, 7) étant appliquée sur la surface de la première phase (5).

28. Dispositif selon la revendication 27, une couche formée à partir du deuxième polymère (3, 8) étant appliquée sur la couche prévue sur la surface.

29. Dispositif selon l'une des revendications précédentes, le marquage étant lié à un corps solide, de préférence un billet de banque.
